# EUROPEAN PATENT SPECIFICATION

(11) **EP 2 244 754 B1**
(45) Date of publication and mention of the grant of the patent: **25.03.2015**
(21) Application number: 09703531.5
(22) Date of filing: 21.01.2009
(51) Int. Cl.: A61L 27/56, A61L 27/58, A61L 27/60

(54) **MULTILAYER SCAFFOLD**
MEHRSCHICHTIGES GERÜST
STRUCTURE MULTICOUCHE

(30) Priority: 25.01.2008 GB 0801405; 15.02.2008 GB 0802767
(43) Date of publication of application: 03.11.2010
(73) Proprietor: Smith & Nephew, PLC, London WC2N 6LA (GB)
(72) Inventor: RAXWORTHY, Michael, John, York YO26 5HT (GB); IDDON, Peter, Damien, York YO41 5PZ (GB); SMITH, Jennifer, Margaret, York YO23 2SY (GB)
(74) Representative: Smith & Nephew
(86) International application number: PCT/GB2009/000165
(87) International publication number: WO 2009/093023

(56) References cited:
- WO-A1-97/06837
- WO-A1-2008/017170
- WO-A1-2008/069759
- WO-A2-2006/106506
- US-A1- 2002 085 994

## Description

### FIELD OF THE INVENTION

The invention relates to biodegradable and/or bioresorbable fibrous articles having utility in medical applications.

### BACKGROUND TO THE INVENTION

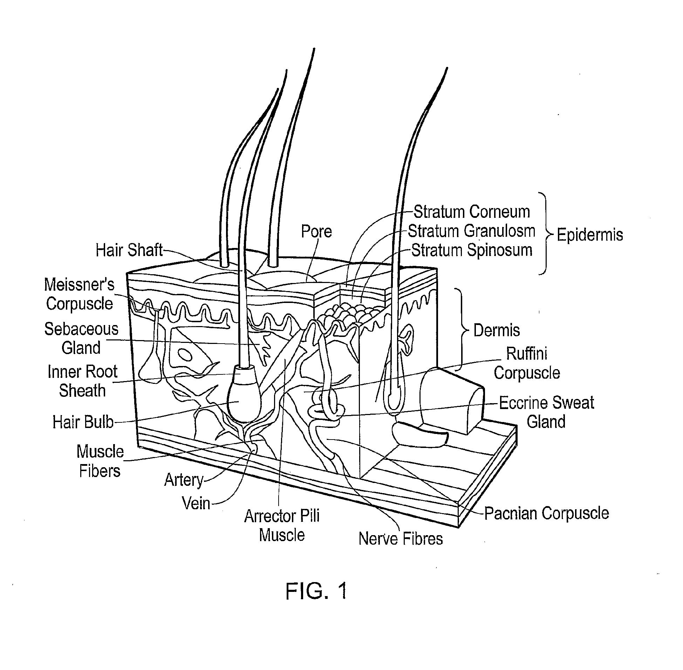

Skin is the largest organ in the body, covering the entire external surface and forming about 8% of the total body mass¹. Skin is composed of three primary layers as illustrated in Figure 1: the *epidermis*, the *dermis,* and the *hypodermis (subcutaneous adipose layer)*.

The epidermis contains no blood vessels, and cells in the deepest layers are nourished by diffusion from blood capillaries extending to the upper layers of the dermis. The main type of cells which make up the epidermis are keratinocytes, with melanocytes and Langerhans cells also present. The dermis provides waterproofing and serves as a barrier to infection.

The dermis is the layer of skin beneath the epidermis that consists of connective tissue and cushions the body from stress and strain. The dermis is tightly connected to the epidermis by a basement membrane. It also harbors many nerve endings that provide the sense of touch and heat.

It contains the hair follicles, sweat glands, sebaceous glands, apocrine glands, lymphatic vessels and blood vessels. The blood vessels in the dermis provide nourishment and waste removal to its own cells as well as the Stratum basale of the epidermis.

Many patients require medical attention following the loss of skin due to accident, illness or surgery. For example, skin cancers can require the excision of areas of full thickness skin. Although most small cancer lesions are sutured following excision, large lesions often cannot be treated in this manner. Larger skin cancers are often referred to a dermatologist or plastic surgeon. In these cases the preferred procedure for plastic surgeons is repair using a skin flap or split-thickness skin graft. This relatively expensive procedure results in a good quality repair, but causes additional morbidity to another body site. Elderly patients or those with complicating medical conditions (e.g. heavy smokers, diabetics) can suffer complications after a graft or flap procedure. These patients can also suffer from poor healing, resulting in repeated visits to a clinician and extended treatment times.

The graft or flap option is not always available to dermatologists, who can either attempt to close the wound by suturing, leave it to heal by secondary intention or refer it to a plastic surgeon. Suturing may not be possible where the excised area is too large, and this upper size limit is reduced in areas of the body where the skin is tighter or scarring is more of a problem (such as the face). Leaving the wound open to heal by secondary intention invites infection and can result in scarring. Referral to a plastic surgeon increases the overall treatment cost and can lead to the potential problems discussed above.

An off-the-shelf regenerative medical device that enabled dermatologists to provide a plastic surgeon-quality repair, without the need for grafts or flaps, would be of significant advantage. Such a device would comprise a scaffold material that assists healing, by allowing the patient's own cells to migrate and proliferate within the damaged area, forming new tissue faster and with fewer complications compared to standard non-surgical interventions.

Numerous other medical procedures or conditions, which result in open wounds, may benefit from the use of this invention. These include, although are not limited to, Mohs surgery, repair of other soft tissue tumours, aesthetic surgery, periodontology, and scar revision surgery.

Existing bioresorbable scaffold technologies are known that facilitate the healing of chronic and acute wounds. A significant number of these technologies exploit the biological properties of relatively pure natural polymers such as collagen, silk, alginate, chitosan and hyaluronate extracted from animal or plant tissue. Examples of these include the collagen matrices produced by Nanomatrix Inc. and the modified cellulose used by Nanopeutics s.r.o.

Other technologies are based upon processed extracellular matrix (decellularised) materials which contain multiple natural macromolecules. One such example is Oasis® (Healthpoint Limited) a biologically derived extracellular matrix-based wound product comprised of acellular porcine small intestinal submucosa (which contains type I collagen, glycosaminoglycans and some growth factors). Another example is the allogeneic/xenogeneic acellular scaffold technology being developed by Tissue Regenix Limited, which is derived from decellularised animal or human tissue.

There are concerns regarding the use of materials derived from natural polymers, due to the potential risk from pathogen transmission, immune reactions, poor mechanical properties and a low degree of control over the biodegradability².

Alternatives to scaffold materials include bioresorbable membranes, such as Suprathel® (PolyMedics Innovations), a freeze-dried copolymer of lactic acid, ε-caprolactone and trimethylene carbonate sold to treat burns. Although potentially bioresorbable, Suprathel® is intended to be removed from wound sites after the wound has healed, so does not act as a bioresorbable scaffold.

The prior art scaffolds are directed towards the repair of a specific layer of skin. For example MySkin^{™} (CellTran Limited) is a cultured autologous epidermal substitute comprising a layer of keratinocytes on a non-bioresorbable silicone sheet.

We have developed a bioresorbable, synthetic scaffold for use in partial or full thickness wounds which has been designed to have an architecture which can be populated by appropriate cell populations and hence regenerate the physiological architecture of the skin. The different component layers of the scaffold are optimised to interact differently with different types of cell, to provide a more directed cell growth compared to a monolayer scaffold material. As cells grow inside the scaffold, the nano/micro-fibres are gradually resorbed by the body.

### SUMMARY OF THE INVENTION

The invention is as claimed in the claims.

According to an aspect of the invention there is provided a bioresorbable, synthetic scaffold comprising at least two fibrous materials.

The first fibrous material comprises pores having a diameter of between 4 µm and 9 µm.

The second fibrous material comprises pores having a diameter of between 0.1 µm and 3.5 µm, or and more particularly between 0.2µm and 2.5µm.

The pore size as herein described can be measured by capillary flow porometry. Capillary flow porometry measures the diameters of through-pores at their most constricted part to give a range of pore diameters for a sample. The pore diameter can be expressed in a number of ways, for example:
*"Largest detected pore diameter"* is the largest pore diameter that the capillary flow porometer can detect in the sample;
*"Diameter at maximum pore size distribution"* provides the pore diameter at the peak of the distribution (i.e. the modal pore size);
*"Mean-flow pore diameter"* provides the median pore diameter.

The scaffold is designed to support the migration and proliferation of human soft tissue cells, such as the cells required to colonise a wound in order for its repair. The different component layers are optimised to interact differently with different cell types, to provide a more directed cell growth compared to a monolayer scaffold material.

In embodiments of the invention first and second fibrous materials are provided as layers which are substantially planar within the scaffold. In particular, these planar layers are adjacent with each other. In such embodiments the scaffold can be considered as a laminate, wherein the scaffold is constructed of different layers of material which are bonded together.

The scaffold is orientated within a wound such that first fibrous material is located beneath the second fibrous material. This orientation encourages fibroblasts to colonise the first fibrous material and keratinoyctes to colonise the second fibrous material, to thereby create the dermis and epidermis, respectively.

The fibroblast is the key cell in the formation of new dermal tissue. It is the principal cell type of the dermal layer of the skin and is responsible for production of extracellular matrix components (ie collagens, fibronectin, elastin, growth factors and cytokines). In intact skin the fibroblast is relatively quiescent and is responsible for the slow turnover of extracellular matrix components. During the wound healing process, however, it differentiates into the myofibroblast and is responsible for the development of mechanical force and hence contributes to wound closure by tissue contraction as well as by deposition of new extracellular matrix to form the basis of granulation tissue to fill the wound space. The myofibroblast is usually lost as repair resolves and is again replaced by the fibroblast on completion of the process of wound remodelling³.

In embodiments of the invention the first layer possesses an optimised architecture to support the migration and proliferation of skin fibroblasts. This enables the recreation of the dermal layer of the skin.

The keratinocyte forms the epidermis, the upper layer of the skin. The epidermis is described as a stratified epithelium and as such, consists of a number of clearly defined layers of keratinocytes from the basal layer adjacent to the basement membrane of the dermis to the *stratum corneum* or cornified layer at the outer surface of the skin. The latter consists of keratinocytes that have completed the process of terminal differentiation to provide the skin with its barrier function and which will eventually be sloughed off as dead cells. Basal keratinocytes cells in contrast, are cells at the beginning of the differentiation process and have significant migratory, proliferative and synthetic properties. They are the cell type responsible for directed migration over newly-repaired dermis to close (or re-epithelialise) a wound and restore barrier function. Keratinocytes form colonies arising originally from a single basal cell and thence sheets of cells as these colonies join. Cells at the leading edge of this sheet migrate from the wound margins to complete wound closure after which terminal differentiation will lead to the formation of a stratified structure. Interactions between fibroblasts and keratinocytes are important to promote and regulate extracellular matrix formation and keratinocyte proliferation⁴.

In embodiments of the invention the second layer possesses an optimised architecture to support the migration and proliferation of human keratinocytes across its surface. This enables the recreation of the epidermal layer of the skin.

The scaffold can be non-woven.

In embodiments of the invention, the first and/or the second layer comprise randomly orientated fibres.

In embodiments of the invention, the first and/or second layer comprise aligned fibres. For example, the fibres can be aligned in a substantially parallel manner.

In embodiments of the invention, the first and/or the second layer comprise microfibres and/or nanofibres.

In embodiments of the invention the fibres in the first fibrous layer have a diameter of 1.2µm to 4.0µm, particularly 1.6µm to 3.4µm and more particularly 2.0µm to 2.8µm.

In embodiments of the invention the fibres in the second fibrous layer have a diameter of 50nm to 1.6µm, particularly 0.1µm to 1.2µm and more particularly 0.2µm to 0.8µm.

The layers of the scaffold are made of any suitable synthetic material which is biocompatible, that is it does not induce adverse effects such as immunological reactions and/or rejections and the like when in contact with the cells, tissues or body fluid of an organism. In embodiments of the invention suitable synthetic fibres include, but are not limited to, aliphatic polyesters, poly(amino acids), copoly(etheresters), polyalkylenes, oxalates, polyamids, tyrosine derived polycarbonates, polyamidoesters, polyoxaesters containing amino groups, poly(anhydrides), polyphosphazenes and combinations thereof.

The use of synthetic materials also avoids the possible risk of disease transmission which may be associated with materials derived from animal or human sources and further avoids the potential ethical and religious barriers to the use of such materials.

It is particularly advantageous that the synthetic material used for first and second layers is biodegradable/bioresorbable. That is, the fibres transiently degrade/resorb within the physiological environment, with the hydrolysis by-products generated during resorption being excreted by normal biochemical pathways. It is particularly advantageous that the scaffold is completely resorbable as this eliminates the need for invasive and painful removal of the scaffold after wound healing is complete.

The first and second layers can be designed to resorb at the same rate or at different rates.

Examples of suitable synthetic, biodegradable/bioresorbable polymers include for example, but are not limited to, polylactic acid (PLA), polyglycolic acid (PGA), polycaprolactone (PCL), polydioxanone (PDO), polytrimethylene carbonate (TMC) and polyethylene glycol (PEG).

The fibres in any one layer of the scaffold can be of the same material.

Alternatively the fibres in any one layer can be of different materials.

The fibres in the first and second layers of the scaffold can be of the same material.

The fibres in the first and second layers can be of different materials.

The thickness of the first and second layer can be varied depending on the depth of the wound. For example, the first and second layer can be of the same thickness. Alternatively, the first layer can be substantially thicker than the second layer, particularly in full-thickness wounds.

The scaffold can comprise at least one further layer. This at least one further layer can have an optimised cell architecture for fibroblasts or keratinocytes or any other cell type involved in wound healing.

In embodiments of the invention additional layers of the scaffold can be added into the wound bed following the absorption of the first and optionally the second layer. This is particularly advantageous as it enables the repair of deeper wounds.

Alternatively the additional layers can be placed into the wound bed either after: (i) a defined amount of time or (ii) a defined amount of regeneration of the dermis and/or epidermis.

At least one of the layers of the scaffold can further comprise active agents which can promote wound healing. For example, agents which improve scar resolution and prevent scar formation, for example insulin, vitamin B, hyaluronic acid, mitomycin C, growth factors, such as TGFβ, cytokines or corticosteroids. These agents can be associated with the fibres, for example attached to the fibres or impregnated within the fibres.

In embodiments of the invention the fibres of the first and/or second layers of the scaffold are electrospun. The technique of electrospinning was first introduced in the early 1930s to fabricate industrial or household non-woven fabric products. In recent years, the technique has been utilised to form scaffolds of polymer fibres for use in tissue engineering. The technique involves forcing a natural or synthetic polymer solution through a capillary, forming a drop of the polymer solution at the tip and applying a large potential difference between the tip and a collection target. When the electric field overcomes the surface tension of the droplet, a polymer solution jet is initiated and accelerated towards the collection target. As the jet travels through the air, the solvent evaporates and a non-woven polymer fabric is formed on the target. Alternatively, the polymer can be electrospun in the form of a melt, where cooling of the jet results in a solid polymer fibre. Such fibrous fabrics, having an average fibre diameter in the micrometre or nanometre scale have been used to fabricate complex three-dimensional scaffolds for use in tissue engineering applications.

The first and second layers can be electrospun separately and then brought into contact with each other. For instance, a surface of the first and second layers can be bonded together to form the scaffold. The bonding can be achieved, for example, by heat treatment, solvent bonding or the use of an adhesive.

Alternatively, one of the layers can form the substrate onto which the other layer is electrospun.

Alternatively the first and second layers can be electrospun as a single unit, with post-formation modification resulting in the layers having different pore architectures. This modification may be based on physical or chemical means, and may for example include selective treatment using heat or a solvent.

It will be known to one skilled in the art of electrospinning that changes can be made to any of the following electrospinning parameters, which will result in scaffolds having differing architectures:
- Electrospinning polymer solution concentration.
- Electrospinning solvent
- Electrospinning voltage
- Electrospinning duration
- Fibre collector type, shape, or construction material
- Diameter, rotation speed or length of cylindrical collector
- Needle traverse distance, frequency or speed
- Needle diameter, length, cross-sectional shape, or construction material
- Number of needles or arrangement of needles
- Needle to collector separation distance
- High voltage configuration
- Solvent conductivity by means of an additive (for example a salt)
- Substrate used to cover fibre collector (including the use of no release paper)
- Ambient atmospheric composition, pressure, temperature or humidity
- Changing any of the conditions above for one or more of the layers to ensure that the solvent has entirely or almost entirely evaporated from the fibres, so that they do not bond together upon impacting on the collector
- Changing any of the conditions above for one or more of the layers to ensure that the solvent is not given sufficient time to substantially evaporate, resulting in partially solvated fibres that partially merge with other fibres on the collector to form highly interconnected porous meshes
- Changing any of the conditions above to an intermediate situation whereby fibres retain enough solvent to allow bonding together with other fibres on the collector without substantially altering the fibrous nature of the scaffolds, to improve scaffold strength and retention of structure

In embodiments of the invention the first fibrous material is placed in the wound bed in order to facilitate dermal repair and regeneration by promoting colonisation by fibroblasts. After a predetermined period of time and/or degree of wound repair, the second fibrous material can be placed above the first fibrous material in order to facilitate epidermal repair and regeneration by promoting the migration of keratinocytes over its upper surface.

In embodiments of the invention, the first fibrous material and the second fibrous material are placed into the wound as a single unit.

In alternative embodiments of the invention the first fibrous material and the second fibrous material are placed into the wound separately. For example, the first fibrous material is placed into the wound for a predetermined period of time and/or until a predetermined degree of dermal regeneration has been achieved. Following this, either one or more additional first fibrous materials can be placed in the wound or the second fibrous material can be placed into the wound.

The fibrous materials can be inserted, either together or separately, into a wound bed in order to promote wound healing.

In embodiments of the invention the first fibrous material possesses an optimised architecture to support the migration and proliferation of skin fibroblasts. This enables the recreation of the dermal layer of the skin.

In embodiments of the invention the second fibrous material possesses an optimised architecture to support the migration and proliferation of human keratinocytes across its surface. This enables the recreation of the epidermal layer of the skin.

In embodiments of the invention the first fibrous material is placed in the wound bed in order to facilitate dermal repair and regeneration by promoting colonisation by fibroblasts. After a predetermined period of time and/or degree of dermal repair has been achieved, the second fibrous material can be placed above the first fibrous material in order to facilitate epidermal repair and regeneration by promoting the migration of keratinocytes over its upper surface.

In embodiments of the invention the kit comprises at least two first fibrous materials. The provision of different sizes of the first fibrous material, in particular the provision of a variety of different thicknesses, enables the use of the first fibrous material to be tailored to an individual wound. For example, a relatively thin first fibrous material can be used in a shallow wound, whereas a relatively thick first fibrous material can be used in deeper wounds. Additional layers of the first fibrous material can be added into the wound bed during the progression of wound repair, thereby allowing the gradual build-up of the dermal layer.

In embodiments of the invention the kit comprises at least two second fibrous materials. The provision of different sizes of the second fibrous material, in particular the provision of a variety of different thicknesses, enables the use of the second fibrous material to be tailored to an individual wound.

In embodiments of the invention the kit further comprises an adhesive, which is used to bond the first and second fibrous materials together.

The method is particularly advantageous for the regeneration of full thickness wounds.

Numerous medical procedures or conditions, which result in open wounds, may benefit from the use of this invention. These include, although are not limited to, Mohs surgery, repair of other soft tissue tumours, aesthetic surgery, periodontology, and scar revision surgery.

The methods can be used to treat humans and non-human animals.

### References

1.Chong E.J et al (2007) "Evaluation of electrospun PCL/gelatine nanofibrous scaffold for wound healing and layered dermal reconstruction. Acta Biomaterialia 3, 321-330.
2. Ma, PX (2004) "Scaffolds for tissue fabrication", Materials Today, 2004, 30-40.
3. Desmouliere A et al (2005) "Tissue repair, contraction and the myofibroblast" Wound Rep Regen 13(1) 7-12.
4. Wemer, S et al (2007) "Keratinocyte-fibroblast interactions in wound healing" J Invest Dermatol 127(5) 998-1008.

The invention will herein be described with reference to the accompanying Examples and Figures, wherein;
Figure 1: Schematic of the architecture of the skin
Figure 2: Schematic of electrospinning method
Figure 3: Scanning electron microscope image of the fibrous PGA scaffold prepared in Example 1. The scale bar corresponds to a length of 5 µm.
Figure 4: Scanning electron microscope image of the fibrous PGA scaffold prepared in Example 2. The scale bar corresponds to a length of 5 µm.
Figure 5: Scanning electron microscope image of the fibrous PGA scaffold prepared in Example 3. The scale bar corresponds to a length of 10 µm.
Figure 6: Scanning electron microscope image of the edge of the fibrous bilayer PGA scaffold prepared in Example 4. The scale bar corresponds to a length of 50 µm.

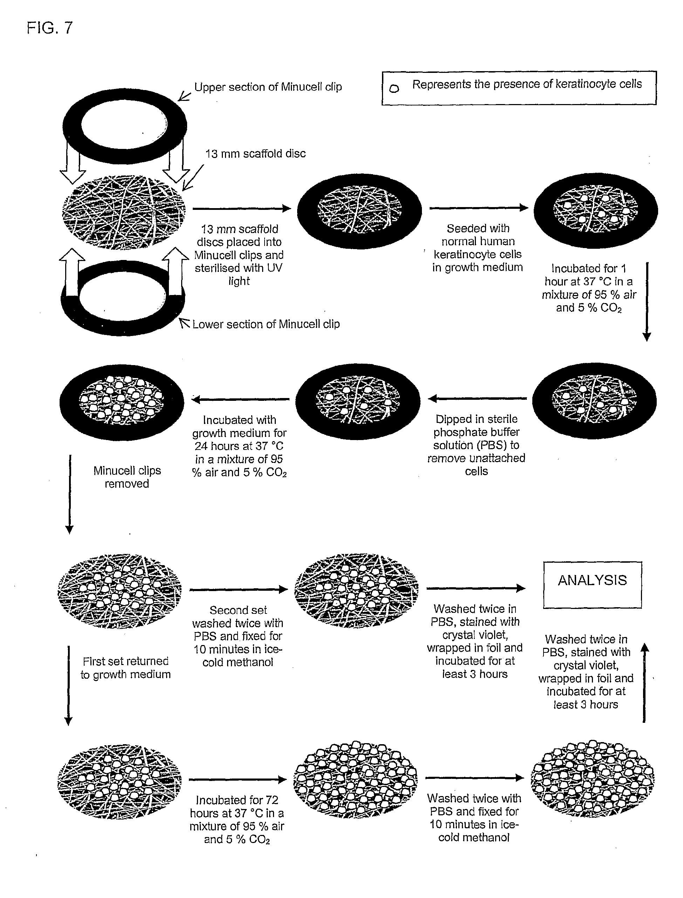

Figure 7: Schematic of the migration assay procedure (not to scale). The representations of keratinocyte cells are for illustrative purposes only, and are not intended to specify actual proliferation behaviour of such cells.
Figure 8: NHEK cells on the scaffold prepared in Example 1 after 24 hours incubation. The left-hand image shows the crystal violet stain under light conditions, the right-hand image shows the DAPI stain in the same field of view under fluorescence conditions. The images were acquired at a magnification of 20.
Figure 9: DAPI-stained NHEK cells on the scaffold prepared in Example 3 after 24 hours incubation. The image was acquired under fluorescence conditions at a magnification of 20.
Figure 10: DAPI-stained NHEK cells on the scaffold prepared in Example 1 after 96 hours incubation. The image was acquired under fluorescence conditions at a magnification of 20. The edge of the scaffold is visible in the top left-hand corner of the image.

### DETAILED EMBODIMENTS OF THE INVENTION

### EXAMPLE 1

A non-woven monolayer scaffold was prepared by electrospinning a solution of poly(glycolic acid) (PGA) in 1,1,1,3,3,3-hexafluoropropan-2-ol (hexafluoroisopropanol, HFIP).

### Solution Preparation

PGA supplied by PURAC Biomaterials (with an approximate weight-average molecular weight of 130,000) was melt-extruded at 260-274 °C using a Rondol Linear 18 single screw extruder and then immediately quenched in water at 5-10 °C. This extruded PGA was used to prepare a 7 w/w % solution in spectrophotometric grade HFIP supplied by Apollo Scientific Ltd (corresponding to a solution viscosity of approximately 0.35 Pa.s). This solution was left rolling overnight at 21°C until dissolved. Prior to electrospinning, the solution of PGA in HFIP was filtered through a 10.0 µm Whatman Polydisc HD filter (polypropylene filter, 50 mm diameter) directly into a 20 mL syringe (polypropylene, lubricant-free, 20.0 mm internal diameter). The resulting polymer solution was free from visible particulates.

In order to increase the conductivity of the polymer solution, a micropipette was used to add 25 w/w % aqueous sodium chloride (NaCl) to the syringe containing the filtered polymer solution, to give a NaCl concentration of 1.0 w/w % relative to the dry weight of PGA in the syringe (assuming a PGA solution density of 1.6 gL⁻¹). After vigorous shaking for 15 minutes, a fine salt precipitate had formed throughout the solution. The syringe was allowed to stand for a further 15 minutes before a final vigorous shake, and was then used for the electrospinning experiments. After the last experiment using this solution, the fine salt precipitate was still well dispersed throughout the solution. All air bubbles were removed from the solution-filled syringe, which was placed into a KD Scientific KDS200 syringe pump (Item 1 in Figure 1) set to dispense at 0.06 mLmin⁻¹ (0.03 mLmin⁻¹ per needle).

### Electrospinning

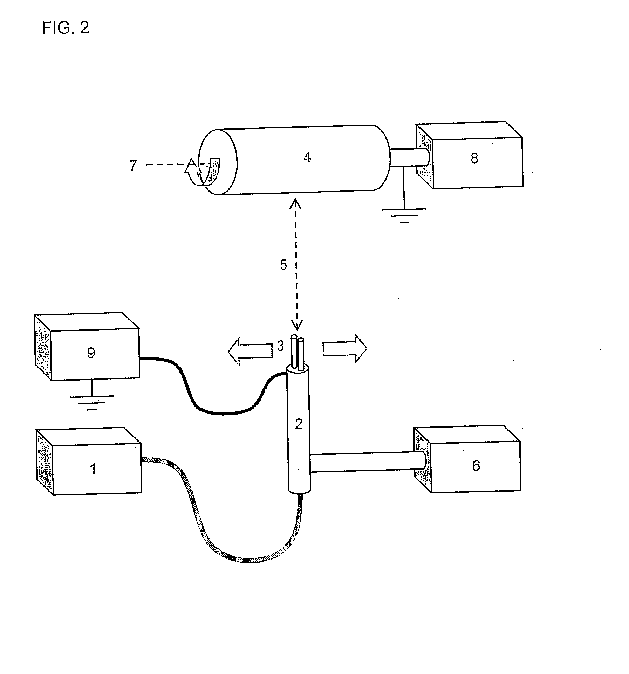

The syringe exit was connected to a HFIP-resistant flexible plastic tube, which then split into two tubes. These tubes connected to two flat-ended 21 gauge steel needles (Item 3 in Figure 2), which were supported in a needle arm (Item 2 in Figure 2) which could be made to traverse by means of a motor (Item 6 in Figure 2). The needles were aligned perpendicularly with respect to the rotational axis (Item 7 in Figure 2) of the earthed 50 mm diameter, 200 mm long steel mandrel (Item 4 in Figure 2), and the needle tip to mandrel separation distance (Item 5 in Figure 2) was set to 150 mm. The needles were set to traverse along the entire 200 mm length of the mandrel, at a rate of one traverse every 18.5 seconds (where a traverse is defined as a single movement forward or backward along the length of the traversing distance).

The mandrel was completely covered in a sheet of non-stick release paper (fastened in place using double-sided adhesive tape) and rotated at 50 rpm by means of a motor (Item 8 in Figure 2). A voltage of 11.0 kV was delivered to the needles (Item 3 in Figure 2) by a Glassman High Voltage Inc. EL50R0.8 High Voltage Generator (Item 9 in Figure 2).

Electrospun fibres were then formed from the PGA solution delivered to the needle tips, and collected on the paper-covered mandrel to form a non-woven scaffold material. Electrospinning was carried out at 21±1 °C. After a period of 60 minutes, the voltage generator was switched off and the scaffold removed from the mandrel. The scaffold was then dried overnight in a vacuum oven at room temperature, to remove any residual HFIP.

### Scaffold Thickness Measurements

The thickness of the single scaffold layer produced was measured at several points along its length (i.e. parallel to the rotational axis of the mandrel) using Mitutoyo Absolute Digimatic digital callipers.

### Scanning Electron Microscopy (SEM)

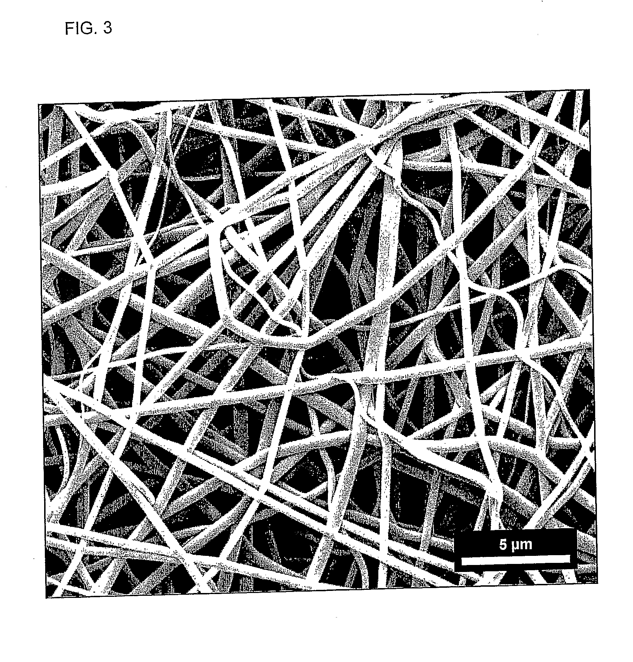

Scaffold samples were attached to 12 mm aluminium SEM stubs using two small pieces of double-sided adhesive to either edge, leaving a central zone without adhesive. The samples were attached so that the upper surface of the scaffold was visible (i.e. the surface deposited towards the end of the experiment). Samples were then sputter coated with gold/palladium alloy to an estimated depth of approximately 30 nm. The coated samples were subsequently imaged by an FEI-Quanta Inspect SEM in the high vacuum mode using a voltage of 5.0 kV and spot diameter of 2.5 nm, in conjunction with FEI Quanta 3.1.1 software. An example SEM image acquired at a magnification of 12,000 is shown in Figure 3.

### Calculation of Mean Fibre Diameter

Three SEM images at a suitable magnification were recorded and printed for one sample of each electrospun fibre scaffold, and these were used to calculate the mean fibre diameter. For each image, the diameters of the first 20 clearly visible fibres along a randomly selected straight line were measured using a ruler. The aggregate 60 measurements from the three images were used to calculate a mean fibre diameter and standard deviation.

### Determination of Pore Diameters

Circular samples (26 mm diameter) were cut from the uniform thickness portion of the scaffolds using a template and scalpel. Capillary flow porometry analysis was carried out on these samples using a PMI Capillary Flow Porometer CFP-1100-AEXL. The wetting fluid used was Galwick (surface tension 15.9 dyn.cm⁻¹) and the test method used was Dry Up/Wet Up with a maximum pressure of 8 or 12 psi.

### Results

Thickness = 100-120 µm across the central 60 % of the scaffold length.
Mean fibre diameter = 0.44 µm ± 0.20 µm.
Largest Detected Pore Diameter = 1.98 µm,
Mean-Flow Pore Diameter (median pore diameter) = 1.11 µm
Diameter at Maximum Pore Size Distribution = 0.93 µm.

### EXAMPLE 2

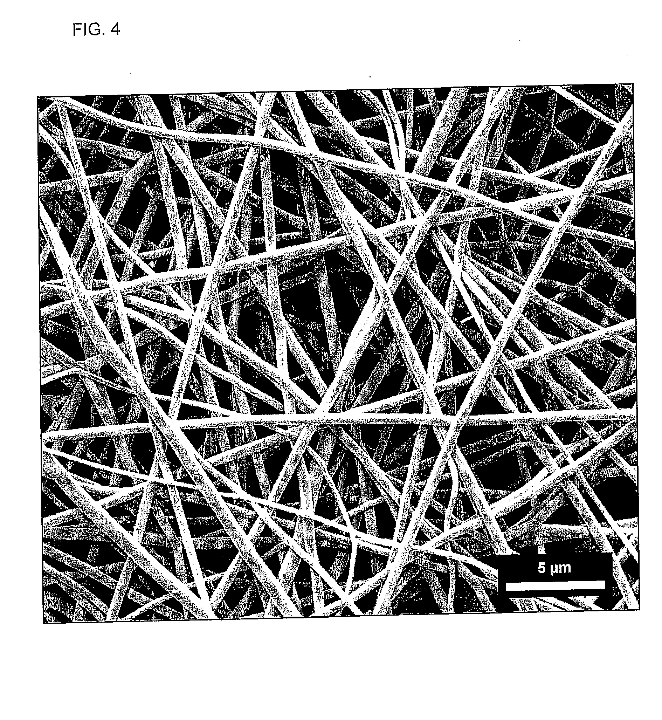

An 8 w/w % solution of PGA in HFIP was prepared and used to prepare a non-woven monolayer scaffold material using the same general method described in Example 1. This concentration of PGA in HFIP corresponds to a solution viscosity of approximately 0.55 Pa.s. Figure 4 shows an SEM image of the scaffold acquired at a magnification of 10,000.

### Results

Thickness = 120-140 µm across the central 65 % of the scaffold length.
Mean fibre diameter = 0.51 µm ± 0.12 µm.
Largest Detected Pore Diameter = 2.29 µm
Mean-Flow Pore Diameter (median pore diameter) = 1.15 µm
Diameter at Maximum Pore Size Distribution = 0.94 µm.

### EXAMPLE 3

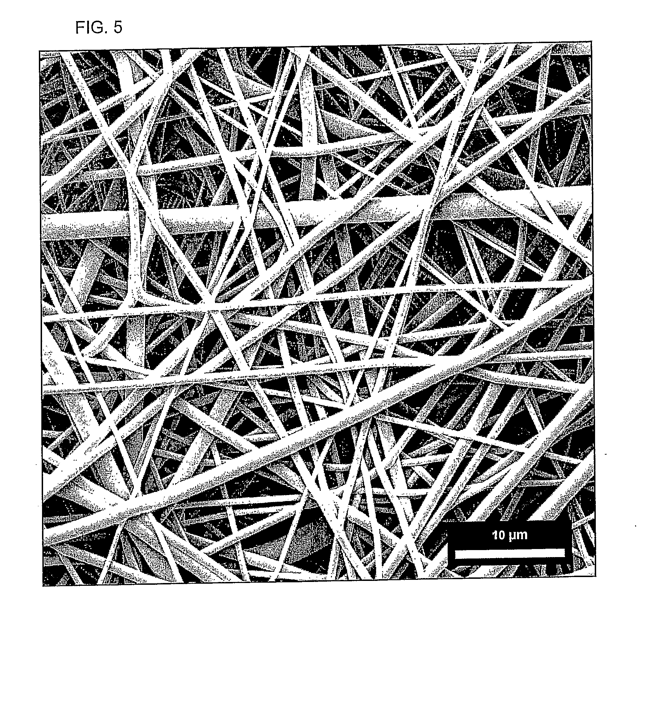

A 9 w/w % solution of PGA in HFIP was prepared and used to prepare a non-woven monolayer scaffold material using the same general method described in Example 1, although no aqueous sodium chloride was added to the solution of PGA in HFIP. This concentration of PGA in HFIP corresponds to a solution viscosity of approximately 0.85 Pa.s. In addition, the electrospinning duration was increased to 68 minutes. Figure 5 shows an SEM image of the scaffold acquired at a magnification of 6,000.

### Results

Thickness = 100-110 µm across the central 70 % of the scaffold length.
Mean fibre diameter = 0.81 µm ±0.38 µm.
Largest Detected Pore Diameter = 3.44 µm
Mean-Flow Pore Diameter (median pore diameter) =1.87 µm
Diameter at Maximum Pore Size Distribution = 1.58 µm.

### EXAMPLE 4

A non-woven bilayer scaffold comprising two layers of different architectures was prepared using 11 w/w % and 8 w/w % solutions of PGA in HFIP, which correspond to solution viscosities of 1.7 Pa.s and 0.55 Pa.s, respectively.

The first layer was prepared using the 11 w/w % solution using the same general method described in Example 1, although no aqueous sodium chloride was added to the solution of PGA in HFIP. In addition, electrospinning duration was decreased to 33 minutes and the mandrel diameter was increased to 150 mm (although the needle to mandrel distance was maintained at 150 mm).

The second layer was prepared using the 8 w/w % solution using the same general method described in Example 1, although no aqueous sodium chloride was added to the solution of PGA in HFIP. This layer was electrospun directly onto the first layer, which had been previously dried overnight in a vacuum oven at room temperature. The electrospinning duration for this layer was 43 minutes.

### Results

### First layer

Thickness = 60-70 µm across the central 75 % of the scaffold length.
Mean fibre diameter = 2.58 µm ± 0.44 µm.

### Second layer

Thickness = 120-130 µm across the central 60 % of the scaffold length.
Mean fibre diameter = 0.68 µm ±0.37 µm.

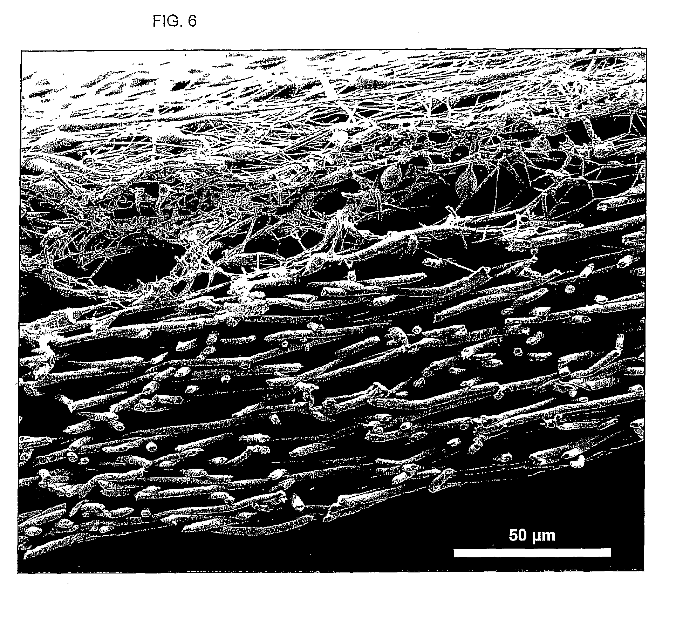

Figure 6 shows an SEM image of the edge of the final bilayer scaffold acquired at a magnification of 1,500.

### Example 5

In order to demonstrate the ability of the second fibrous material layer to support the migration and proliferation of keratinocytes, the *in vitro* migration behaviour of human keratinocyte cells on the scaffolds prepared in Examples 1 to 3 was evaluated. These scaffolds were compared to two positive controls: Thermanox coverslips (supplied by Nunc GmbH); and a 100-110 µm thick electrospun PGA scaffold with a larger mean fibre diameter of 2.46 µm (S.D. 0.50 µm), prepared using the same general method described in Example 1 (although using an 11 w/w% solution of PGA in HFIP). This latter scaffold is similar to those described in WO 07/132186 (to Smith and Nephew) which has been demonstrated to support fibroblast migration and proliferation.

### Migration Assay

The scaffolds and controls were cut into 13 mm diameter discs using a Samco SB-25 Hydraulic Press, placed into Minucell clips (part number 1300, Minucell and Minutissue Vertriebs, GmbH) and sterilised under UV light for 20 minutes using an Amersham UV Cross-Linker. Normal human keratinocyte cells (NHEK; supplied by Promocell GmbH) were seeded onto the discs in 100 µl of Keratinocyte Growth Medium (KGM-2; Promocell GmbH) at a density of 100,000 cells per disc and allowed to adhere for one hour at 37 °C in a 95 % air and 5 % CO₂ mixture. After one hour, the discs were dipped in sterile phosphate buffer solution (PBS) to remove any unattached cells, and placed into the wells of a 24 well plate containing 2 ml of KGM-2 medium. The resulting discs were incubated for 24 hours at 37 °C in a 95 % air and 5 % CO₂ mixture.

After 24 hours, the Minucell clips were removed. The first set of discs was returned to the plate containing KGM-2 medium and incubated for a further 72 hours. The second set was washed twice with PBS, and fixed for 10 minutes in ice-cold methanol. The methanol was then removed and the discs washed twice more with PBS. 0.5ml of crystal violet stain (0.1 % in PBS; supplied by Sigma-Aldrich Ltd) was added to each disc. The plate was then wrapped in foil to prevent the stain from photo-bleaching, and incubated at room temperature for a minimum of three hours. After a total incubation time of 96 hours, the first set of discs were stained using an identical method.

The schematic shown in Figure 6 illustrates this procedure.

### Analysis

Since keratinocytes migrate as colonies on one plane, migration was assessed visually rather than by quantifying cell numbers. After incubation, the discs were washed twice with PBS and mounted onto glass slides using mounting medium containing 4',6-diamidino-2-phenylindole (DAPI; supplied by Vector Laboratories Ltd). Slides were then visualised using a Leica DMLB Fluorescent Microscope.

Table 1 shows the observations for keratinocyte migration on the scaffolds and controls for the 24 hour and 96 hour time points. *"Clear inner edge"* indicates that the cells migrated over the available scaffold surface up to the edge of the white (inner) Minucell clip and formed an inner circle of cells. *"Cells at outer edge"* indicates that the cells moved away from this inner circle towards the outer perimeter of the scaffold, and partially reached the outer edge of the scaffold. *"Cells at outer edge all way around"* indicates that the cells migrated from the inner edge and were visible around the entire outer edge of the scaffold (i.e. covered the entire scaffold surface).

Migration occurred on all the scaffolds and the Thermanox coverslips, however it is clear that the best migration for keratinocytes occurred on the scaffolds possessing the smallest fibre diameters (Example 1 [7 w/w %] and Example 2 [8 w/w %]).

**TABLE 1**

| Scaffold | Mean Fibre Diameter (µm) | Incubation Time | |
|---|---|---|---|
| | | 24 hours | 96 hours |
| Thermanox Control (sample 1) | N/A | Clear inner edge | Cells at outer edge |
| Scaffold Control (sample 1) | 2.46 | Clear inner edge | Cells at outer edge (signs of scaffold degradation) |
| Example 1 (sample 1) | 0.44 | Clear inner edge | Cells at outer edge all way around |
| Example 1 (sample 2) | 0.44 | Clear inner edge | Cells at outer edge all way around |
| Example 1 (sample 3) | 0.44 | Clear inner edge | Cells at outer edge all way around |
| | | | |
| Thermanox Control (sample 2) | N/A | No clear inner edge | Cells at outer edge |
| Scaffold Control (sample 2) | 2.46 | Clear inner edge | Cells at outer edge all way around (signs of scaffold degradation) |
| Example 2 (sample 1) | 0.51 | No clear inner edge (lots of stain) | Cells at outer edge all way around |
| Example 2 (sample 2) | 0.51 | Clear inner edge | Cells at outer edge all way around |
| Example 2 (sample 3) | 0.51 | Clear inner edge | Cells at outer edge all way around |
| | | | |
| Thermanox Control (sample 3) | N/A | No clear inner edge | Cells at outer edge |
| Scaffold Control (sample 3) | 2.46 | Clear inner edge | Cells at outer edge (signs of scaffold degradation) |
| Example 3 (sample 1) | 0.81 | Clear inner edge | Cells at outer edge |
| Example 3 (sample 2) | 0.81 | Clear inner edge | Cells at outer edge |
| Example 3 (sample 3) | 0.81 | Clear inner edge | Cells at outer edge |

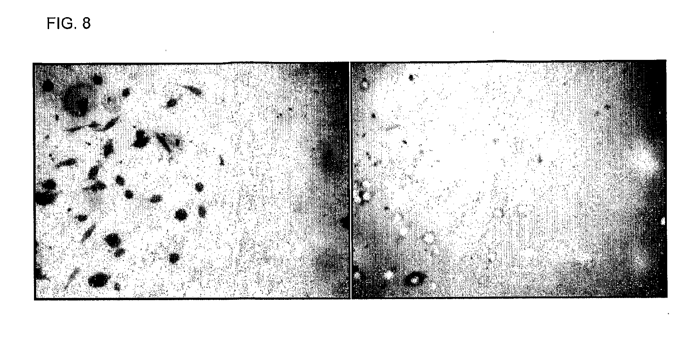

Figure 8 shows NHEK cells on the scaffold prepared in Example 1 after 24 hours incubation. The two images are the same field of view visualised under light conditions to show the crystal violet stained cells (left-hand side), and under fluorescence conditions to show the DAPI stained cells (right-hand side). These images show that the crystal violet is staining the cells, and not the background scaffold. The boundary edge of the area left uncovered during incubation is clearly visible down the centre of each image.

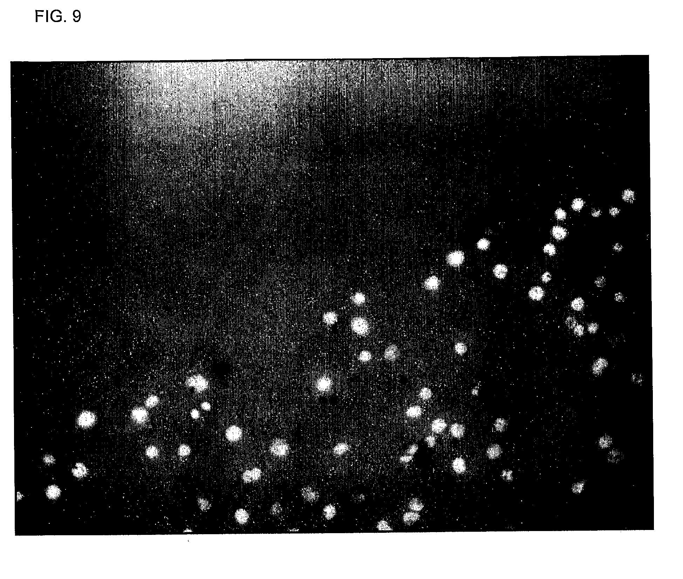

Figure 9 shows a typical example of NHEK cells on the scaffold prepared in Example 3 after 24 hours incubation. The cells were stained using DAPI and visualised under fluorescence conditions. The boundary edge of the area left uncovered during incubation is clearly visible running from the bottom left-hand corner of the image to the top right-hand corner. A clear edge to this area shows that the cells had attached to the scaffold and have filled the area available to them, but have not yet been able to infiltrate the area of scaffold covered by the Minucell clip. After 96 hours incubation, the scaffolds were stained and visualised on the fluorescent microscope. Preliminary signs of degradation were observed for the control scaffolds: some broken fibres were visible, which were beginning to take up the crystal violet and DAPI stains. However, this did not affect the ability to distinguish keratinocyte cells from the scaffold material.

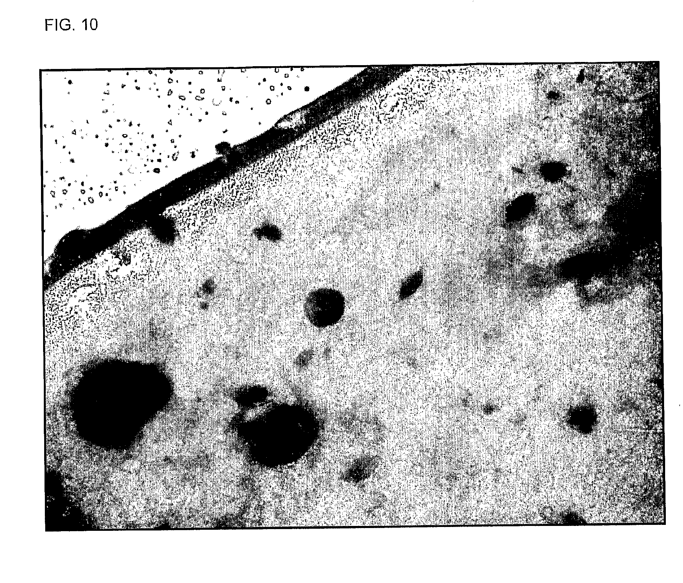

Figure 10 shows a typical example of NHEK cells on the scaffold prepared in Example 1 after 96 hours incubation. The cells have migrated to the edge of the scaffold, which is visible in the top left-hand corner. The cells are visible all around the scaffold edge. Similar results were obtained for the scaffold prepared in Example 2.

The NHEK cells on the control scaffold after 96 hours incubation were not visible all around the scaffold edge, and were present in fewer numbers. The scaffold prepared in Example 3 behaved similarly to the control scaffold.

The conclusions drawn from these Examples are:
- NHEK cells adhere to all the electrospun scaffolds and are visible on the scaffold surfaces after 24 hours incubation.
- NHEK cells migrate to the edges of all the scaffolds within 96 hours incubation.
- The two scaffolds prepared in Examples 1 and 2 supported NHEK cell migration better that the scaffold control evidenced by the distance covered by the migrating edge of the keratinocyte sheet. This is due to the different architectures (Examples 1 and 2 possessed smaller mean fibre diameters and pore sizes).
- The scaffold prepared in Example 3 behaved in a similar manner to the scaffold control, as it had a larger mean fibre diameter and pore size compared to Examples 1 and 2.

## Claims

1. A bioresorbable, synthetic scaffold comprising at least two fibrous materials, **characterised in that** the first fibrous material comprises pores having a diameter of between 4µm and 9µm and the second fibrous material comprises pores having a diameter of between 0.1µm and 3.5µm, and wherein, in use, the first fibrous material is located beneath the second fibrous material, **characterised in that** the scaffold is for use in skin regeneration.

2. A scaffold according to claim 1, wherein the first fibrous material and the second fibrous material are made of different composition.

3. A scaffold according to claim 1 or 2, wherein the at least two fibrous materials form layers within the scaffold.

4. A scaffold according to claim 3, wherein the layers are substantially planar.

5. A scaffold according to claims 3 or 4, wherein the layers are adjacent with each other.

6. A scaffold according to any of claims 2 to 5, wherein the scaffold is a laminate comprising a layer of a first fibrous material bonded to a layer of a second fibrous material, and wherein the first and second materials are made of a different composition

7. A scaffold according to any preceding claim, wherein the fibrous materials are non-woven.

8. A scaffold according to any preceding claim, wherein at least one of the first fibrous material and the second fibrous material are electrospun.

9. A kit comprising a bioresorbable synthetic scaffold as claimed in claim 1.

## Patentansprüche

1. Ein bioresorbierbares synthetisches Gerüst, das mindestens zwei faserige Materialien beinhaltet, **dadurch gekennzeichnet, dass** das erste faserige Material Poren mit einem Durchmesser von zwischen 4 µm und 9 µm beinhaltet und das zweite faserige Material Poren mit einem Durchmesser von zwischen 0,1 µm und 3,5 µm beinhaltet, und wobei sich im Gebrauch das erste faserige Material unterhalb des zweiten faserigen Materials befindet, **dadurch gekennzeichnet, dass** das Gerüst zur Verwendung bei der Hautregeneration dient.

2. Gerüst gemäß Anspruch 1, wobei das erste faserige Material und das zweite faserige Material aus einer unterschiedlichen Zusammensetzung hergestellt sind.

3. Gerüst gemäß Anspruch 1 oder 2, wobei die mindestens zwei faserigen Materialien Schichten innerhalb des Gerüsts bilden.

4. Gerüst gemäß Anspruch 3, wobei die Schichten im Wesentlichen ebenflächig sind.

5. Gerüst gemäß den Ansprüchen 3 oder 4, wobei die Schichten nebeneinander liegen.

6. Gerüst gemäß einem der Ansprüche 2 bis 5, wobei das Gerüst ein Schichtstoff ist, beinhaltend eine Schicht aus einem ersten faserigen Material, das mit einer Schicht aus einem zweiten faserigen Material verbunden ist, und wobei das erste und das zweite Material aus einer unterschiedlichen Zusammensetzung hergestellt sind.

7. Gerüst gemäß einem der vorhergehenden Ansprüche, wobei die faserigen Materialien Vliesstoff sind.

8. Gerüst gemäß einem der vorhergehenden Ansprüche, wobei mindestens eines von dem ersten faserigen Material und dem zweiten faserigen Material elektrogesponnen ist.

9. Ein Kit, das ein bioresorbierbares synthetisches Gerüst gemäß Anspruch 1 beinhaltet.

## Revendications

1. Un échafaudage synthétique biorésorbable comprenant au moins deux matières fibreuses, **caractérisé en ce que** la première matière fibreuse comprend des pores ayant un diamètre compris entre 4 µm et 9 µm et la deuxième matière fibreuse comprend des pores ayant un diamètre compris entre 0,1 µm et 3,5 µm, et dans lequel, lors de l'utilisation, la première matière fibreuse est située sous la deuxième matière fibreuse, **caractérisé en ce que** l'échafaudage est destiné à être utilisé dans la régénération de la peau.

2. Un échafaudage selon la revendication 1, dans lequel la première matière fibreuse et la deuxième matière fibreuse sont réalisées en une composition différente.

3. Un échafaudage selon la revendication 1 ou la revendication 2, dans lequel les au moins deux matières fibreuses forment des couches au sein de l'échafaudage.

4. Un échafaudage selon la revendication 3, dans lequel les couches sont substantiellement planes.

5. Un échafaudage selon les revendications 3 ou 4, dans lequel les couches sont adjacentes l'une à l'autre.

6. Un échafaudage selon n'importe lesquelles des revendications 2 à 5, l'échafaudage étant un stratifié comprenant une couche d'une première matière fibreuse liée à une couche d'une deuxième matière fibreuse, et dans lequel les première et deuxième matières fibreuses sont réalisées en une composition différente.

7. Un échafaudage selon n'importe quelle revendication précédente, dans lequel les matières fibreuses sont non tissées.

8. Un échafaudage selon n'importe quelle revendication précédente, dans lequel soit la première matière fibreuse, soit la deuxième matière fibreuse est électrofilée.

9. Un kit comprenant un échafaudage synthétique biorésorbable tel que revendiqué dans la revendication 1.
